(19) Europäisches Patentamt
European Patent Office
Office européen des brevets

(11) Veröffentlichungsnummer: **0 325 915 A2**

(12) **EUROPÄISCHE PATENTANMELDUNG**

(21) Anmeldenummer: **89100122.4**

(22) Anmeldetag: **05.01.89**

(51) Int. Cl.4: **C07C 147/12 , C08G 18/10 , C08G 18/38 , C08G 18/32**

(30) Priorität: **16.01.88 DE 3801083**

(43) Veröffentlichungstag der Anmeldung: **02.08.89 Patentblatt 89/31**

(84) Benannte Vertragsstaaten: **AT BE CH DE ES FR GB IT LI NL SE**

(71) Anmelder: **BAYER AG**
**D-5090 Leverkusen 1 Bayerwerk(DE)**

(72) Erfinder: **Heise, Klaus-Peter, Dr.**
**Am Gartenfeld 74**
**D-5068 Odenthal(DE)**
Erfinder: **Grögler, Gerhard, Dr.**
**von-Diergardt-Strasse 48**
**D-5090 Leverkusen 1(DE)**
Erfinder: **Wedemeyer, Karlfried, Dr.**
**Bilharzstrasse 7**
**D-5000 Köln 80(DE)**
Erfinder: **Dieterich, Dieter, Dr.**
**H. T.-von-Boettinger-Strasse 16**
**D-5090 Leverkusen 1(DE)**

(54) **Diaminoarylsulfone, Verfahren zu deren Herstellung und Verwendung bei der Herstellung von Polyurethanelastomeren.**

(57) Die vorliegende Erfindung betrifft neue Diaminoarylsulfone der Formel (I)

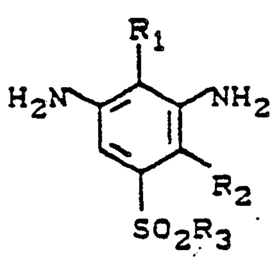

(I)

in der $R_1$, $R_2$ und $R_3$ unabhängig voneinander einen Alkylrest mit 1 bis 12 C-Atomen bedeuten, ein Verfahren zur Herstellung dieser neuen Verbindungen und deren Verwendung zur Herstellung von Polyurethanelastomeren.

EP 0 325 915 A2

## Diaminoarylsulfone, Verfahren zu deren Herstellung und Verwendung bei der Herstellung von Polyurethanelastomeren

Die vorliegende Erfindung betrifft neue Diaminoarylsulfone der Formel (I)

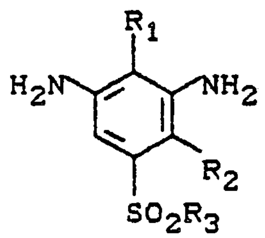

(I)

in der $R_1$, $R_2$ und $R_3$ unabhängig voneinander einen Alkylrest mit 1 bis 12 C-Atomen bedeuten, ein Verfahren zur Herstellung dieser neuen Verbindungen und deren Verwendung.

Bei der Herstellung von Polyurethanelastomeren spielen aromatische Diamine eine wichtige Rolle. Sie ermöglichen den Einsatz von TDI-Prepolymeren, die gute Lagerstabilität und günstige Viskosität im Gießverfahren aufweisen.

Wichtigstes technisches Diamin ist 3,3′-Dichlor-4,4′-diaminodiphenylmethan (MBOCA), das jedoch gewisse Nachteile aufweist. Seit langem werden daher Ersatzstoffe gesucht und es sind inzwischen auch eine Reihe von Produkten bekannt geworden, die sich mit TDI-Prepolymeren zu Elastomeren umsetzen lassen (vgl. z.B. C. Hepburn, Polyurethane Elastomers, Appl. Sci. Publ. Ltd., London, New York (1982), Seiten 101-138.

Von diesen hat jedoch nur der in der Deutschen Auslegeschrift 1 803 635 beschriebene 3,5-Diamino-4-chlor-benzoesäure-isobutylester in der Praxis Bedeutung erlangt.

Diese Verbindung weist wie bisher alle zu einem guten Werteniveau führenden langsamen aromatischen Diamine, eine symmetrische Struktur auf und verbindet ein sehr gutes Verarbeitungsverhalten mit hohem Qualitätsniveau der daraus erhältlichen Elastomeren.

Nachteilig ist indessen das relativ aufwendige Herstellungsverfahren, vor allem die besondere Vorsichtsmaßnahmen erfordernde Dinitrierung.

Es wurde nun überraschenderweise gefunden, daß die erfindungsgemäßen Diaminoarylsulfone sehr gute Eignung als Diamin-Kettenverlängerungsmittel bei der Herstellung von Elastomeren, insbesondere nach dem Gießverfahren, aufweisen. In ganz besonderem Maß gilt dies für 1,3-Diamino-4,6-dimethyl-5-methylsulfonyl-benzol.

Die erfindungsgemäßen Diaminoarylsulfone ergeben PUR-Elastomere mit einem den Produkten gemäß DE-AS 1 803 635 ebenbürtigen Werteniveau, haben jedoch den Vorteil, daß sie einfacher herzustellen sind und insbesondere die Dinitrostufe keine besonderen Vorsichtsmaßnahmen erfordert. Die Reaktivität ist etwas höher als diejenige des genannten Esterdiamins, was im Hinblick auf maschinelle Gießverfahren und für den Einsatz im RIM-Prozeß vorteilhaft ist.

Die vorliegende Erfindung betrifft neue sulfongruppenhaltige alkylsubstituierte aromatische Diamine, in denen die Aminogruppe in m-Stellung zur Sulfongruppe stehen. Die erfindungsgemäßen sulfongruppenhaltigen aromatischen Diamine entsprechen der Formel (I)

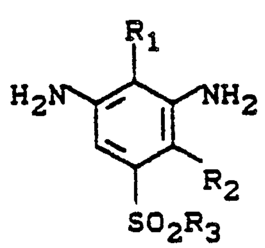

(I)

in der $R_1$, $R_2$ und $R_3$ unabhängig voneinander einen Alkylrest mit 1 bis 12 C-Atomen, bevorzugt 1 bis 6 C-Atomen, bedeutet. Besonders bevorzugt werden Verbindungen der Formel (I), in denen $R_1$ und $R_2$ für den Methyl-Rest stehen.

Gegenstand der vorliegenden Efindung ist ferner ein Verfahren zur Herstellung der neuen Verbindungen. Die Herstellung der neuen, Sulfongruppen aufweisenden aromatischen Diamine kann in Anlehnung an bekannten Verfahren erfolgen. So erhält man z.B. Diamine der allgemeinen Formel (I), in dem man entsprechende Sulfone der Formel (II)

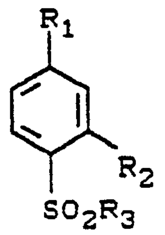

(II)

in der $R_1$, $R_2$ und $R_3$ die gleiche Bedeutung wie in Formel (I) haben, dinitriert und anschließend in an sich bekannter Weise, z.B. durch katalytische Hydrierung oder Eisenreduktion, reduziert.

Die Sulfone der Formel (II) sind teils bekannt wie z.B. 1,3-Dimethyl-4-methylsulfonyl-benzol oder lassen sich auf bekannten Wegen, beispielsweise durch Oxidation aus Sulfiden oder Sulfoxiden oder durch Alkylieren von entsprechenden Sulfinsäure-Salzen herstellen. Die Herstellung von Sulfonen ist z.B. in Houben Weyl, Bd. IX, 1955, auf Seite 227 bis 241 beschrieben.

Als Beispiele für die neuen erfindungsgemäßen sulfongruppenhaltigen alkylsubstituierten aromatischen Diamine der Formel (I) seien genannt:

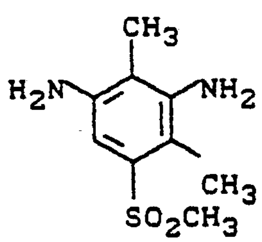

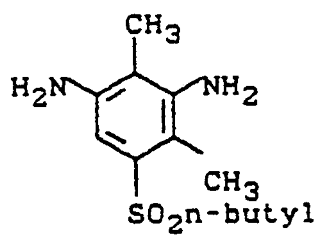

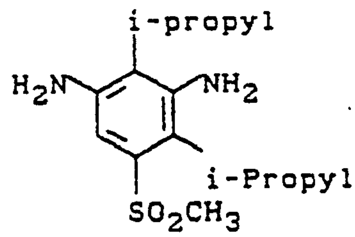

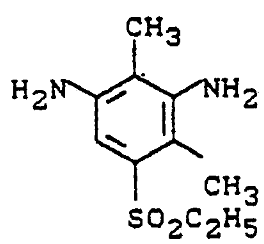

Als besonders wertvoll erweisen sich die Verbindungen der Formel (I) als Kettenverlängerungsmittel bei der Herstellung von Kunststoffen mit kautschukelastischen Eigenschaften nach dem bekannten Isocyanat-Polyadditionsverfahren.

Die vorliegende Erfindung betrifft somit auch ein Verfahren zur Herstellung von Polyurethanelastomeren aus Verbindungen mit mindestens 2 Hydroxylgruppen vom Molekulargewicht 800 bis 5.000, Diisocyanaten und aromatischen Diaminen als Kettenverlängerungsmittel, welches dadurch gekennzeichnet ist, daß als Kettenverlängerungsmittel die erfindungsgemäßen Diamine der Formel (I) ein gesetzt werden. Als Ausgangsmaterial für das erfindungsgemäße Verfahren kommen neben den erfindungsgemäßen aromatischen Diaminen Verbindungen mit mindestens 2 Hydroxylgruppen vom Molgewicht 800 bis 5.000, vorzugsweise vom Molekulargewicht 1.000 bis 3.000, in Frage; beispielsweise lineare oder schwach verzweigte Hydroxylgruppen aufweisende Polyester wie sie z.B. in an sich bekannter Weise aus di- oder höherfunktionellen Alkoholen und Carbonsäuren oder Oxycarbonsäuren, gegebenenfalls unter Mitverwendung von Aminoalkoholen, Diaminen, Oxyaminen und Diaminoalkoholen nach bekannten Verfahren hergestellt werden können.

Diese Polyester können Doppel- oder Dreifachbindungen von ungesättigten Fettsäuren enthalten, in Frage kommen auch lineare oder schwach verzweigte Polyether, wie sie durch Polymerisation von Alkylenoxiden wie Ethylenoxid, Propylenoxid, Epichlorhydrin oder Tetrahydrofuran gewonnen werden können. Auch Mischpolymerisate dieser Art können verwendet werden. Geeignet sind ferner durch Anlagerung der genannten Alkylenoxide an z.B. polyfunktionelle Alkohole, Aminoalkohole oder Amine herstellbare lineare oder verzweigte Anlagerungsprodukte. Als polyfunktionelle Startkomponenten für die Addition der Alkylenoxide seien beispielsweise genannt: Ethylenglykol, 1,2-Propylenglykol, Hexandiol-(1,6), Ethanolamin und Ethylendiamin; trifuktionelle Startkomponenten wie Trimethylolpropan oder Glycerin können anteilweise mitverwendet werden. Selbstverständlich können auch Gemische linearer und/oder schwach verzweigter Polyalkylenglykolether verschiedenen Typs eingesetzt werden. Erfindungsgemäß als Ausgangsmaterial zu verwendende Diisocyanate können beliebiger Art sein.

Als Diisocyanate seien z.B. die aliphatischen Diisocyanate der allgemeinen Formel

$OCN-(CH_2)_n-NCO$

$n = 2$ bis 8

cycloaliphatische Diisocyanate wie Hexahydrotoluylendiisocyanat-(2,4) und -(2,6) bzw. Gemische dieser Isomeren oder Dicyclohexylmethandiisocyanat, araliphatische Diisocyanate wie 1,3-Xylylendiisocyanat und aromatische Diisocyanate wie Toluylendiisocyanat-(2,4) oder -(2,6) und Gemische dieser Isomeren. Phenylendiisocyanat-(1,4), Diphenylmethandiisocyanat-(4,4′), Diphenyletherdiisocyanat-(4,4′) oder Naphthylendiisocyanat-(1,5) genannt. Erfindungsgemäß kommen als Diisocyanate auch Isophorondiisocyanat sowie Esterdiisocyanate von Carbonsäuren in Frage, wie sie z.B. in der britischen Patentschrift 965 474 beschrieben werden. Triisocyanate können anteilmäßig mitverwendet werden.

Die Mengen an Reaktionskomponenten werden in der Regel so gewählt, daß das Molverhältnis von Diisocyanaten zu Kettenverlängerer plus Verbindung mit reaktionsfähigen OH-Gruppen, welches vom jeweils angewendeten Verarbeitungsverfahren abhängt, in der Regel zwischen 1,0 und 1,5 liegt, vorzugsweise zwischen 1,05 und 1,25.

Das Molverhältnis von $NH_2$-Gruppen des Kettenverlängerers zu reaktionsfähigen OH-Gruppen kann in weiten Grenzen variieren, vorzugsweise soll es zwischen 0,4 und 1,5 liegen, wobei weiche bis harte Typen resultieren.

Die Durchführung des erfindungsgemäßen Anwendungsverfahrens kann auf verschiedene Weise erfolgen. So kann man z.B. die Verbindung mit mindestens 2 Hydroxylgruppen mit einem überschuß an Diisocyanat zur Reaktion bringen und nach der Zugabe des erfindungsgemäßen Kettenverlängerungsmittels die Schmelze in Formen gießen. Nach mehrstündigem Nachheizen ist ein hochwertiger elastischer Polyurethankunststoff entstanden.

Eine weitere Ausführungsform besteht darin, daß man die höhermolekulare Verbindung mit mindestens zwei Hydroxylgruppen im Gemisch mit dem erfindungsgemäß zu verwendenden Kettenverlängerungsmittel mit einem Überschuß an Diisocyanat umsetzt und das Reaktionsprodukt nach der Granulierung in der Hitze unter Druck verformt. Je nach den angewendeten Mengenverhältnissen der Reaktionsteilnehmer können hierbei Polyurethankunststoffe mit verschiedenartigen Härten und verschiedenartiger Elastizität erhalten werden. Auf diese Weise entstehen Kunststoffe, die sich wie Thermoplaste verarbeiten lassen. Eine weitere Ausführungsform besteht darin, daß man die höhermolekulare Verbindung mit mindestens zwei Hydroxylgruppen im Gemisch mit dem erfindungsgemäß zu verwendenden Kettenverlängerungsmittel mit einem Unterschuß an Diisocyanat umsetzt, wobei ein walzbares Fell erhalten wird, das in anschließender Stufe z.B. durch Vernetzung mit weiterem Diisocyanat, in einen kautschukelastischen Polyurethankunststoff überführt werden kann.

Bevorzugt werden in das erfindungsgemäße Anwendungsverfahren Diamine der Formel (I) eingesetzt, in denen $R_1$, $R_2$ und $R_3$ für einen Alkylrest mit 1 bis 6 C-Atomen steht. Besonders bevorzugt werden Verbindungen der Formel (I), in denen $R_1$ und $R_2$ für den Methylrest stehen, als Kettenverlängerungsmittel in das erfindungsgemäße Verfahren eingesetzt.

Ganz besonders bevorzugt ist die Verbindung 3,5-Diamino-1-methylsulfonyl- 2,4-dimethyl-benzol als Kettenverlängerungsmittel.

In den DE-OS 2 001 772 und DE-OS 2 025 896 ist bereits vorgeschlagen worden, 3,5-Diamino-1-ethylsulfonyl-4-chlor-benzol als Kettenverlängerungsmittel zur Herstellung von PUR-Elastomeren einzusetzen. Derartige Halogensulfone weisen jedoch im Vergleich sowohl zu den analog strukturierten Halogenbenzoesäureestern als auch im Vergleich zu den erfindungsgemäßen Produkten einen gravierenden Nachteil auf: es handelt sich um sehr reaktionsträge Substanzen. Selbst bei erhöhter Temperatur beträgt die bis zur Entformung erforderliche Zeit mehrere Stunden. Dabei ist das mechanische Werteniveau trotz der symmetrischen Struktur nur mittelmäßig.

Es muß im Lichte dieses Standes der Technik also umso überraschender angesehen werden, daß mit den erfindungsgemäßen von Chloratomen freien und unsymmetrischen Sulfonen sowohl ein günstiges Verarbeitungsverhalten und eine rasche Aushärtung als auch ein hervorragendes mechanisches Werteniveau erzielt werden kann.

Der Gegenstand der vorliegenden Erfindung soll nun anhand der folgenden Beispiele noch näher erläutert werden.

## Herstellung von Ausgangsverbindungen

### a) 2,4-Dimethyl-5-methylsulfonyl-1,3-dinitro-benzol

138,5 g (0,734 Mol) 2,4-Dimethyl-1-methylsulfonylbenzol (GC 97,5 %ig; Beilstein H 5, S. 491) werden in 200 ml Schwefelsäuremonohydrat vorgelegt und mit 118 g (1,83 Mol) 98 %iger Salpetersäure dinitriert. Bei 20 bis 30 °C wird unter äußerer Kühlung die Hälfte der Salpetersäure zugetropft, dann läßt man die Temperatur auf 70 °C ansteigen und tropft die restliche Menge zu. Nach 30 minütigem Nachrühren bei 70 °C wird das Reaktionsgemisch auf 700 ml Eiswasser gegeben, abgenutscht und mit Wasser, verdünnter Natriumhydrogencarbonat-Lösung und nochmals Wasser gewaschen.

Nach Trocknen im Vakuum verbleiben 180 g der Dinitroverbindung, die laut GC weniger als 0,3 % Mononitroverbindung enthält.
Schmelzpunkt 175 °C (Toluol), $^1$H-NMR ($CDCl_3$): δ - 2,53 (S, 3H, aromat.-$CH_3$), 2,69 (S, 3H, aromat-$CH_3$), 3,17 (S, 3H, -$SO_2$-$CH_3$) und 8,65 ppm (S, 1H, aromat. H).

### b) 2,4-Dimethyl-5-ethylsulfonyl-1,3-dinitro-benzol

100 g (0,5 Mol) 1-Ethylsulfonyl-2,4-dimethyl-benzol (Beilstein H 5, S. 491) werden nach der vorstehenden Vorschrift mit 83,6 g (1,3 Mol) 98 %iger Salpetersäure dinitriert:
138 g Dinitroverbindung (92 % der Theorie), Gehalt (GC) 99,6 %, Schmelzpunkt (Ethanol) 150 bis 151 °C.

### c) 2,4-Dimethyl-5-propylsulfonyl-1,3-dinitro-benzol

Analog a) wurde 2,4-Dimethyl-1-propylsulfonylbenzol (Beilstein H 5, S. 491) dinitriert:
Schmelzpunkt 139 bis 141 °C, $^1$H-NMR ($CDCl_3$: δ - 1,07 (T, J = 7 Hz, 3H, -$CH_2$-$CH_2$-$\underline{CH_3}$), 1,80 (M, J = 7 Hz, 2 H, $CH_2$-$\underline{CH_2}$-$CH_3$), 2,51 (S, 3H, aromat.-$CH_3$), 2,67 (S, 3H, arom.-$CH_3$), 3,13 (M, 2H, -$\underline{CH_2}$-$CH_2$-$CH_3$) und 8,58 ppm (S, 1H, aromat. H).

### d) 5-Isopropylsulfonyl-2,4-dimethyl-1,3-dinitro-benzol

Analog a) wurde 1-Isopropylsulfonyl-2,4-dimethyl-benzol dinitriert:
Schmelzpunkt 138,5 °C (Ethanol), $^1$H-NMR (DMSO): δ = 1,21 (D, J = 7 Hz, 6H, -$CH_2$-($\underline{CH_3}$)$_2$), 2,45 und 2,60 (je S, je 3H, aromat. -$CH_3$), 3,64 (M, J = 7 Hz, 1H, -$\underline{CH}$-($CH_3$)$_2$) und 8,50 ppm (S, 1H, aromat. H).

### e) 5-Butylsulfonyl-2,4-dimethyl-1,3-dinitro-benzol

Analog a) wurde 1-Butylsulfonyl-2,4-dimethyl-benzol dinitriert:
Schmelzpunkt 116 bis 118 °C, $^1$H-NMR ($CDCl_3$): δ = 0,94 (T, J = 7 Hz, 3H, -$CH_2$-$CH_3$), 1,46 (6 Linien, J = 7 Hz, 2H,-$CH_2$-$CH_2$-$\underline{CH_2}$-$CH_3$) 1,75 (M, 2H, -$CH_2$-$\underline{CH_2}$-$CH_2$-$CH_3$), 2,52 (S, 3H, aromat. -$CH_3$), 2,67 (S, 3H, aromat. -$CH_3$), 3,16 (M, 3H, -$\underline{CH_2}$-$CH_2$-$CH_2$-$CH_3$) und 8,58 ppm (S, 1H, aromat. H).

### f) 2,4-Diisopropyl-5-methylsulfonyl-1,3-dinitro-benzol

Analog a) wurde 2,4-Diisopropyl-1-methyl-sulfonyl-benzol dinitriert. Zur Vervollständigung der Dinitrierung wurde nach Zugabe der Salpetersäure noch 20 %iges Oleum (1,6 Mol) $SO_3$ pro Mol Sulfon) zugesetzt und die Nachrührzeit auf 60 Stunden verlängert. Das Rohprodukt wird zum Entfernen geringer Mengen Mononitroverbindung mit Essigester ausgekocht.

Schmelzpunkt 214 bis 215 °C, $^1$H-NMR ($CDCl_3$): δ = 1,33 und 1,37 (je D, J = 7 Hz, je 6H, -CH-($\underline{CH_3}$)$_2$,

2,95 (M, J = 7 Hz, 1H, -CH-$(CH_3)_2$), 3,18 (S, 3H, $-SO_2-CH_3$), 4,8 (breit, 1H, -CH-$(CH_3)_2$) und 8,25 ppm (S, 1H, aromat. H).

## Beispiel 1

### 1,3-Diamino-2,4-dimethyl-5-methylsulfonyl-benzol

104 g (0,379 Mol) 2,4-Dimethyl-5-methylsulfonyl-1,3-dinitro-benzol werden mit 150 ml Isopropanol in einem 0,7 l-VA-Autoklav in Gegenwart von 6 g wasserfeuchtem Raney-Nickel bei 110 °C und einem Gesamtdruck von 10 bar hydriert. Es wird heiß vom Kontakt abfiltriert. Beim Erkalten des Filtrats auf Raumtemperatur scheidet sich das Diamin kristallin ab: 69,2 g (85 % der Theorie), Schmelzpunkt (Isopropanol) 131 °C, Gehalt (GC) 99,9 %. $^1$H-NMR (DMSO): δ = 1,90 (S, 3H, aromat. $-CH_3$), 2,25 (S, 3H, aromat. $-CH_3$), 3,01 (S, 3H, $-SO_2-CH_3$), 4,67 und 4,83 (je S, je 2H, $-NH_2$) und 6,63 ppm (S, H, aromat. H).

Durch Einengen kann aus der Mutterlauge restliches Diamin gewonnen werden.

## Beispiel 2

### 1,3-Diamino-5-ethylsulfonyl-2,4-dimethyl-benzol

Analog Herstellungsbeispiel 1 wurde 1-Ethylsulfonyl-2,4-dimethyl-3,5-dinitro-benzol zum Diamin hydriert:

Schmelzpunkt (Ethanol) 155 °C, $^1$H-NMR ($CCl_4$): δ - 1,25 (T, J = 7 Hz, 3H, $-CH_2-CH_3$), 2,02 (S, 3H, aromat. $-CH_3$), 2,40 (S, 3H, aromat. $-CH_3$), 3,00 (Q, 2H, $-CH_2-CH_3$), 3,7 (breit, 4H, $-NH_2$) und 6,85 ppm (S, 1H, aromat. H).

## Beispiel 3

### 1,3-Diamino-2,4-dimethyl-5-propylsulfonyl-benzol

Analog Herstellungsbeispiel 1 wurde 2,4-Dimethyl-1,3-dinitro-5-propylsulfonyl-benzol zum Diamin hydriert:

Schmelzpunkt (Isopropanol) 159 bis 160 °C, $^1$H-NMR ($CDCl_3$): δ = 0,98 (T, J = 7 Hz, 3H, $-CH_2-CH_2-CH_3$), 1,72 (M, J = 7 Hz, 2H, $-CH_2-CH_2-CH_3$). 2,02 (S, 3H, aromat. $-CH_3$), 2,38 (S, 3H, aromat. $-CH_3$), 3,02 (M 2H, $-CH_2-CH_2-CH_3$); 3,62 und 3,72 (je S, breit, je 2H, $-NH_2$) und 6,84 ppm (S, 1H, aromat. H).

## Beispiel 4

### 1,3-Diamino-5-isopropylsulfonyl-2,4-dimethyl-benzol

Analog Herstellungsbeispiel 1 wurde 1-Isopropylsulfonyl-2,4-dimethyl-2,5-dinitro-benzol zum Diamin reduziert:

Schmelzpunkt (Isopropanol) 143,5 bis 145 °C, $^1$H-NMR ($CDCl_3$): δ = 1,29 (D, J = 7 Hz, 6H, -CH-$(CH_3)_2$), 2,04 (S, 3H, aromat. $-CH_3$), 2,40 (S, 3H, aromat. $-CH_3$), 3,18 (7 Linien, J = 7 Hz, 1H, -CH-$(CH_3)_2$), 3,65 (S, breit, 4H, $-NH_2$) und 6,84 ppm (S, 1H, aromat. H).

## Beispiel 5

### 1,3-Diamino-5-butylsulfonyl-2,4-dimethyl-benzol

Analog Herstellungsbeispiel 1 wurde 1-Butylsulfonyl-2,4-dimethyl-3,5-dinitro-benzol zum Diamin reduziert:
Schmelzpunkt (Ethanol) 121 bis 122° C, $^1$H-NMR ($CDCl_3$): δ = 0,89 (T, J = 7 Hz, 3H, $-CH_2-CH_3$), 1,36 (6 Linien, J = 7 Hz, 2H, $-CH_2-CH_3$), 1,66 (M, 2H, $-CH_2-CH_2-CH_2-CH_3$), 2,02 (S, 3H, aromat. $-CH_3$), 2,40 (S, 3H, aromat. $-CH_3$), 3,03 (M, 2H, $-CH_2-CH_2-CH_2-CH_3$), 3,58 (S, breit, 4H, $-NH_2$) und 6,84 ppm (S, 1H, aromat. H).

Beispiel 6

1,3-Diamino-2,4-diisopropyl-5-methylsulfonyl-benzol

Analog Herstellungsbeispiel 1 wurde 2,4-Diisopropyl-5-methylsulfonyl-1,3-dinitro-benzol zum Diamin hydriert:
Schmelzpunkt (Toluol) 164 bis 165° C, $^1$H-NMR ($CDCl_3$): δ = 1,38 (D, J - 7 Hz, 6H, $-CH-(CH_3)_2$), 1,42 (D, J = 7 Hz, 6H, $-CH-(CH_3)_2$), 3,02 (S, 3H, $-SO_2-CH_3$), 3,19 (M, J = 7 Hz, 1H, $-CH-(CH_3)_2$), 3,65 und 3,90 (S, breit, je 2H, $-NH_2$), 4,04 (M, J = 7 Hz, 1H, $-CH-(CH_3)_2$) und 6,78 ppm (S, 1H, aromat. H).

Anwendungsbeispiele 7-11

Die Verarbeitung der in nachstehender Tabelle angeführten Diaminoalkylphenyl-sulfone erfolgt im Gießverfahren. In den angegebenen Beispielen wurden die entsprechenden Diamine in geschmolzener Form einem geeigneten NCO-end-gruppenenthaltenden Polyaddukt zugemischt. Als NCO-Prepolymer wurde ein nach bekanntem Verfahren hergestelltes Additionsprodukt aus 2,1 Mol (365,4 g) 2,4-Diisocyanatotoluol (TDI) und 1 Mol(2.000 g) eines linearen Polyesters aus Adipinsäure und Ethylenglykol (OH-Zahl: 56, MG 2.000) mit einem NCO-Gehalt von 3,90 % verwendet. Jeweils 500 g des genannten NCO-Prepolymers wurden vor der Verarbeitung ca. 15 Minuten bei 80° C im Wasserstrahlvakuum entgast. Danach erfolgte die Zugabe des geschmolzenen Diamins in der Menge, daß ein vollständiger (äquimolarer) Umsatz der NCO-Gruppen des Prepolymers mit den $NH_2$-Gruppen des Diamins gegeben ist ($NCO/NH_2$-Verhältnis = 1:0). Die Gießzeit der Reaktionsansätze bei 70 bis 80° C wird hierbei von der jeweiligen Reaktivität der erfindungsgemäßen Diamine bestimmt. Im allgemeinen liegt diese in dem von der Praxis geforderten Bereich von 2 bis 10 Minuten. Der Reaktionsansatz wird in eine auf ca. 100° C erwärmte und mit Trennmittel versehenen Form gegossen und 1/2 bis 4 Stunden bei 120° C ausgeheizt. Nach der Entformung (1/2 bis 1 Stunde) wurden die PUR-Elastomere noch 10 bis 12 Stunden bei 120° C getempert.

Tabelle 1 ergibt einen Überblick über die Gießzeit der Reaktionsansätze und die mechanischen Eigenschaften der aus den erfindungsgemäßen Diaminen erhaltenen Elastomeren.

Tabelle 1

| Beispiel | 7 | 8 | 9 | 10 | 11 |
|---|---|---|---|---|---|
| $R_1$ | $CH_3$ | $CH_3$ | $CH_3$ | $CH_3$ | $CH_3$ |
| $R_2$ | $CH_3$ | $CH_3$ | $CH_3$ | $CH_3$ | $CH_3$ |
| $R_3$ | $CH_3$ | $C_2H_5$ | $C_3H_7$ | $CH(CH_3)_2$ | $C_4H_9$ |
| Gießzeit (min) | 4 | 5 | 3 | 6 | 6 |
| Modul 100 % (MPa) | 6,2 | 5,1 | 4,2 | 2,2 | 4,5 |
| Menge/g pro 500 g NCO-Prep. | 49,7 | 52,6 | 57,6 | 56,2 | 59,4 |
| Zugfestigkeit (MPa) | 37,7 | 27,8 | 20,0 | 17,6 | 21,4 |
| Bruchdehnung (%) | 652 | 650 | 650 | 550 | 700 |
| Weiterreißfestigkeit (KN/m) | 75,0 | 65,2 | 26,0 | 57,8 | 61,1 |
| Härte Shore A | 86 | 85 | 84 | 78 | 82 |
| Elastizität (%) | 34 | 27 | 25 | 23 | 27 |

Beispiel 12 (Stand der Technik; nicht erfindungsgemäß)

Zu 500 g des in den Beispielen 7 bis 11 genannten NCO-Prepolymeren mit einem NCO-Gehalt von 3,9 % werden 54,4 g geschmolzenes Ethyl-(3,5-diamino-4-chlorphenylsulfon der Formel

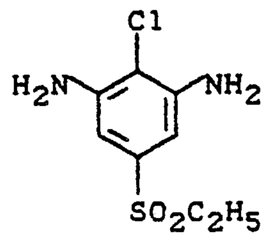

zugemischt. Die Verarbeitungsbedingungen zur Herstellung eines Polyurethan-Elastomeren erfolgte wie bereits angegeben. Der Reaktionsansatz verhält sich im Vergleich zu den bisher beschriebenen Versuchen im Verfestigungsbild jedoch völlig andersartig. Es erfolgt auch bei einer Reaktionstemperatur von 70 bis 80 °C nur eine sehr langsame Umsetzung des Diamins mit den NCO-Gruppen des Prepolymers. Die Gießzeit des Ansatzes beträgt ca. 45 bis 60 °C. Aber auch die Verfestigungszeit der flüssigen Ansätze ist bei 120 °C sehr lang, so daß die Probekörper erst nach mehreren Stunden (3 bis 5 Stunden) entformt werden konnten. Bei den in Beispiel 7 bis 11 beschriebenen Versuchsansätzen beträgt die Entformungszeit nur 15 bis 60 Minuten, was eine zügige Verarbeitung der beiden Komponenten erlaubt.

Man erhält ein Polyurethanharnstoff-Elastomer mit folgenden mechanischen Eigenschaften

| | |
|---|---|
| Modul bei 100 %: | 2,9 MPa |
| Zugfestigkeit: | 17,9 MPa |
| Bruchdehnung: | 650 % |
| Weiterreißfestigkeit: | 37 KN/m |
| Härte Shore A: | 83 |
| Elastizität: | 21 % |

**Ansprüche**

1. Diaminoarylsulfone der Formel (I)

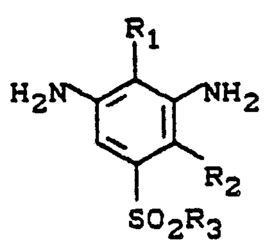

(I)

in der $R_1$, $R_2$ und $R_3$ unabhängig voneinander einen Alkylrest mit 1 bis 12 C-Atomen bedeuten.

2. Diaminoarylsulfone der Formel (I) nach Anspruch 1, dadurch gekennzeichnet, daß $R_1$, $R_2$ und $R_3$ für einen Alkylrest mit 1 bis 6 C-Atomen stehen.

3. Diaminoarylsulfone der Formel (I) nach einem der Ansprüche 1 oder 2, dadurch gekennzeichnt, daß $R_1$ und $R_2$ für den Methylrest stehen.

4. Verfahren zur Herstellung von Diaminoarylsulfonen der Formel (I) gemäß einem der Ansprüche 1 bis 3, dadurch gekennzeichnet, daß man Sulfone der allgemeinen Formel

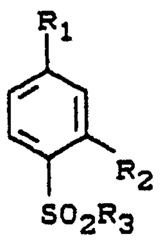

(II)

in der $R_1$, $R_2$ und $R_3$ die gleiche Bedeutung wie in Anspruch 1 haben, dinitriert und anschließend die entsprechende Dinitroverbindung reduziert.

5. Verwendung von Verbindungen gemäß einem der Ansprüche 1 bis 3 als Kettenverlängerungsmittel bei der Herstellung von Polyurethanelastomeren aus Verbindungen mit mindestens zwei Hydroxylgruppen vom Molgewicht 800 bis 5.000, Diisocyanaten und aromatischen Diaminen.